(19) Europäisches Patentamt
European Patent Office
Office européen des brevets

(11) Publication number: **0 232 034 B1**

(12) **EUROPEAN PATENT SPECIFICATION**

(45) Date of publication of patent specification: **08.07.92**

(51) Int. Cl.[5]: **C12N 15/16**, C12N 15/63

(21) Application number: **87300399.0**

(22) Date of filing: **19.01.87**

(54) **Production of human erythropoietin.**

(30) Priority: **23.01.86 JP 12868/86**

(43) Date of publication of application:
**12.08.87 Bulletin 87/33**

(45) Publication of the grant of the patent:
**08.07.92 Bulletin 92/28**

(84) Designated Contracting States:
**CH DE FR GB LI**

(56) References cited:
**EP-A- 0 148 605**
**GB-A- 2 085 887**

(73) Proprietor: **SUMITOMO CHEMICAL COMPANY, LIMITED**
**Kitahama 4-chome 5-33**
**Chuo-ku Osaka 541(JP)**

(72) Inventor: **Yanagi, Hideki**
**14-26, Hanayashiki-Matsugaoka**
**Takarazuka-shi Hyogo 665(JP)**
Inventor: **Ogawa, Ikuzo**
**2-10-3-355, Sonehigashimachi**
**Toyonaka-shi Osaka 561(JP)**
Inventor: **Okamoto, Minoru**
**15-10-204, Kusunokicho**
**Ashiya-shi Hyogo 659(JP)**
Inventor: **Hozumi, Tatsunobu**
**2-10-3-315, Sonehigashimachi**
**Toyonaka-shi Osaka 561(JP)**
Inventor: **Soga, Ayuko**
**2-57-403, Hamamatsubaracho**
**Nishinomiya-shi Hyogo 662(JP)**
Inventor: **Yoshima, Tadahiko**
**2-10-3-357, Sonehigashimachi**
**Toyonaka-shi Osaka 561(JP)**
Inventor: **Tsutsumi, Masahiro**
**1295-110, Shidehara**
**Sanda-shi Hyogo 669-13(JP)**

(74) Representative: **Cresswell, Thomas Anthony et al**
**J.A. Kemp & Co. 14 South Square Gray's Inn**
**London WC1R 5EU(GB)**



Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

## Description

The present invention relates to production of human erythropoietin.

Human erythropoietin is a glycoprotein of a molecular weight of approximately 36,000 daltons mainly produced in kidney. It is a hormone regulating the production of red blood cells by acting on committed stem cells (committed to give raise to red blood cells) to stimulate the differentiation of the cells into proerythroblasts, thereby maintaining normal numbers of red blood cells, or erythrocytes.

When living bodies become anemia, a homeostatic mechanism works to increase the erythropoietin concentration in the blood depending upon the degree of the anemia to cure the anemia, However, in the case of anemia caused by chronic renal deficiency, erythropoietin is not produced because of the destruction of the kidney function and hence the erythropoietin concentrations in the blood do not increase.

Such renal anemia can be treated by administering erythropoietin to supplement an insufficiency of erythropoietin of the patients. Thus, erythropoietin can be used as a therapeutic agent for renal anemia.

As a production method for erythropoietin, there have so far been known a method of isolating erythropoietin from urine of patients with aplastic anemia (J. Biol. Chem., vol. 252, P. 5558 (1977), Japanese patent publication (Kohai) No. 59-155395), a method of purifying erythropoietin from plasma of anemic animals (e.g. sheep) (Pro. Nct. Acad. Sir. USA. vol. 68, P. 697 (1971)), and a method of purifying erythropoietin from culture media of human renal carcinoma or hepatoma cells (Endocrinol vol. 99, P. 504 (1976), Japanese patent publication (Kokai) Nos. 54-55790, 60-16929, and 60-43395).

However, the use of plasma of anemic animals as a source of erythropoietin gives raise to an antigenicity problem considering the fact that erythropoietin is administered as a therapeutic agent to human being.

Since it is difficult to obtain large amounts of urine of patients with aplastic anemia, and the yields of erythropoietin by the culture of human renal carcinoma or hepatoma cells are quite low, production of a large amount of purified erythropoietin has been difficult.

For the large scale production or purification of physiologically active proteins naturally occuring in very small amounts like erythropoietin, recombinant DNA technology provides very useful and advantageous methods. Namely, such physiologically active proteins can be produced in a large scale by introducing the DNA sequences corresponding to the amino acid sequences of physiologically active proteins into host cells such as E. coli, yeasts, animal or plant cells and culturing the resulting transformants to express the desired substances. In this process, the DNA sequences to be introduced may be either genomic DNA, cDNA syntherized from mRNA or chemically manufactured DNA sequences encoding the desired substances.

Today, it is considered that the large scale production of erythropoietin can only be performed by means of recombinant DNA technology. For the production of erythropoietin by recombinant DNA technology, it is necessary to use as host cells, animal cells which can add sugar chains to polypeptides, because the sugar chains are essential to exhibit erythropoietin activity in vivo.

K. Jacobs et al. have reported the cloning of the human erythropoietin cDNA from a cDNA library prepared from human fetal liver and the nucleotide sequence and expression of the erythropoietin cDNA (Nature vol. 313, P. 806 (1985)). F. Lin et al. have also reported the cloning of the human erythropoietin gene from a genomic library and the sequencing and expression of the gene (PCT international publication No. WO85/2610, Pro. Nat. Acad. Sci. USA vol. 82, P. 7580 (1985)).

In the former report, it is described that the human erythropoietin cDNA was inserted into a plasmid containing the SV40 origin of replication, which was then introduced into COS cells derived from the monkey kidney, and that the expression of human erythropoietin in COS cells was confirmed. In this method, the plasmids containing the human erythropoietin cDNA were not integrated into the chromosomes of the COS cells, but replicated autonomously in the cells to several tens of thousands of copies. As a result, the host cells died and hence the expression of human erythropoietin was just transient.

In the latter report, it is described that, in addition to COS cells, Chinese hamster ovary (CHO) cells defective in the enzyme dihydrofolate reductase, DHFR,were used as host cells. In this method, the human erythropoietin genomic DNA ligated to the SV40 late promoter was transfected together with the gene encoding DHFR into the CHO dhfr$^-$ cells and the human erythropoietin gene was integrated along with the DHFR gene into the chromasomes of the host CHO cells. The resulting cells were further selected to give the cells which acquired the resistance to methotrexate (MTX), DHFR-inhibiting agent, due to an amplication of the DHFR gene. Along with the amplification of the DHFR gene, the human erythropoietin gene was amplified, resulting an increased expression of erythropoietin. Thus, the cell lines which permanently produce human erythropoietin in large amounts can be obtained.

The method of increasing expression of a given gene in host CHO dhfr$^-$ cells by increasing the resistance of the cells to MTX, thereby amplifying the gene in the host cells, has been widely known (U.S. Patent 4,399,216). For example, this method was used for the production of tissue plasminogen activator by recombinant DNA technology (Japanese patent publication (Kokai) No. 59-183693).

Although, as mentioned above, it is possible to increase expression level using, as host cells, CHO dhfr$^-$ cells and it is considered that such increased expression will permanently continue, there are still various technical difficulties, particularly in the large scale cultivation of CHO cells partly because CHO cells are anchorage dependent. Thus, it has not yet been possible to produce human erythropoietin in sufficient amounts.

Although CHO cells have an ability to add sugar chains to polypeptides, it has not yet certain that the sugar chain structures of expression products of an introduced human gene in CHO cells are identical to those of naturally-occuring substances. In fact, it is reported that the sugar chain structure of the expression product was different from that of the naturally-occuring erythropoietin when the human erythropoietin was produced in CHO cells (PCT international publication number WO 85/2610).

In the case of glycoproteins to be administered as therapeutic agents, it is undesirable for the glycoproteins to have sugar chains different in structure from the naturally-occuring ones because it might be a cause of antigenicity to recipients.

In order to solve these problems, the present inventors have extensively studied on the introduction and expression of human erythropoietin gene in human cells, in particular Namalwa cells in considering that the most suitable host cells for the production of human erythropoietin by recombinant DNA technology are human cells and that the large scale cultivation of these cells can be conducted relatively easily. As a result, we have succeeded in efficiently expressing human erythropoietin in Namalwa cells. Based on this success, the present invention was accomplished.

The method of the large scale cultivation of Namalwa cells, which grow rapidly and which can be cultured in suspension, has already been established. In fact large-scale production of interferon is being carried out by cultivation of Namalwa cells. Since Namalwa cells are human cells, they are capable of adding native human sugar chains to polypeptides.

The present invention provides transformed Namalwa cells, capable of producing human erythropoietin effectively, and a method for producing human erythropoietin by cultivating said transformed human cells.

In the present invention, the human erythropoietin gene to be introduced into the host human cells may be either the genomic DNA or cDNA coding for human erythropoietin.

To construct functional human erythropoietin gene in transcription as well as translation in Namalwa cells, one or more promoters functional in high eucaryotic cells such as the promoter of human erythropoietin gene, promoters of viruses such as SV 40 and the like are ligated to the 5'-end of the DNAs coding for human erythropoietin, and a polyadenylation site and a splice site derived from, for example, human erythropoietin gene, SV40 early gene of $\beta$-globin gene are ligated to the 3'-end.

Enhancer sequences effective in human lymphocytes may be ligated in the vicinity of the human erythropoietin gene in order to increase the efficacy of the transcription of the gene. As such enhancer sequence, there may be exemplified the enhancers of SV40 or adenovirus, the enhancers found in the region called long terminal repeat of retrovirus or the enhancers of immunoglobulin gene.

It is a usual practice to introduce selective marker genes such as neomycin resistance gene or xanthine-guanine phosphoribosyl transferase gene together with genes coding for desired proteinaceous substances into host cells in order to efficiently select transformed cells. In the present invention, the selection of desired transformants can effectively be conducted by introducing the said selective marker gene together with human erythropoietin gene into Namalwa cells.

As for the method for introducing DNA into Namalwa cells, 1) a method of introducing DNA into host cells after forming a complex of the DNA with calcium phosphate (Virology vol. 52, P. 456 (1973)); 2) a method of introducing DNA into host cells after combining the DNA with DEAE-dextran (Proc. Nat. Acad. Sci. USA,vol. 78, P.7575 (1981)); 3) a method of introducing DNA by fusion of host cells with protoplasts of E. coli carrying the plasmid containing a desired DNA (Proc. Nat. Acad. Sci. USA,vol. 80, P. 825 (1983)), and 4) a method of introducing a desired DNA into host cells by electric pulse (Proc. Nat. Acad. Sci. USA, vol. 81, P. 7161 (1984)).

The Namalwa cells to be used in the present invention are available from, for example, American Type Culture Collection (e.g., ATCC CRL 1432).

The Namalwa cells transformed to have the ability to produce human erythropoietin as above can be grown in culture medium usually used for cultivation of animal cells. For example, RPMI 1640 supplemented with 5 - 10 % of fetal bovine serum can preferably be used.

For the cultivation of the Namalwa cells capable of producing human erythropoietin, there can be applied a method of suspension culture using flasks or dishes usually used for tissue culture, a method of growing the cells in suspension by stirring the culture medium in spinner vessels, a method of growing the cells in hollow fibers into which culture medium is continuously circulated or a method of growing the cells in jar fermentors for cultivation of animal cells.

After the cultivation of the Namalwa cells, the human erythropoietin secreted into the culture medium can be purified by known methods, for example, the method of Miyake et al. (J. Biol. Chem. vol. 252, P. 5558 (1977)) or the method of Yanagawa et al. (J. Biol. Chem. vol. 259, P. 2707 (1984)) after the cells were removed by centrifugation or filtration.

The human erythropoietin gene used in the present invention can be isolated from human renal carcinoma or hepatoma cells producing human erythropoietin by known methods (Nature vol. 313, P. 806 (1985); PCT international publication WO 85/2610; Pro. Nat. Acad. Sci. USA,vol. 82, P. 7580 (1985)).

The nucleotide sequence of human erythropoietin cDNA used in the present invention and the deduced amino acid sequence are shown in Fig. 1. The 27 amino acids of the N-terminal of the amino acid sequence starting from the methionine and ending at the glycine of the Fig. 1 are considered to be a signal sequence for secretion of human erythropoietin which consists of 166 amino acids, starting from the alanine as shown in Fig. 1.

Example:

The following example are given to illustrate the present invention more precisely, but not intended to limit the scope of the present invention thereto. It is to be understood that the present invention encompasses not only the specifically disclosed examples but also any modifications, variations or equivalents thereof.

Isolation of human erythropoietin cDNA

The human erythropoietin cDNA shown in Fig. 1 was isolated in the following manner:

A cDNA library was prepared by the Okayama-Berg method (Mol. Cell. Biol. vol. 2, P. 161 (1982)) from the poly (A) RNA, which was isolated from the erythropoietin-producing human hepatoma cell Hp-1, available from JIKKEN DOBUTSU CHUO KENKYUSHO (Central Institute for Experimental Animals), Japan, by the guanidium thiocyanate method (Biochemistry vol. 18, P. 5294 (1979)), and oligo(dT)-cellulose column chromatography (Proc. Nat. Acad. Sci. USA,vol. 69, P. 1408 (1972)).

The DNA with the following sequence:

5'CAGCAGGGCCAGGCCCTGCCACACTTCCACGGCCTGCTGG
CCCACTTCCAT3'

which is complementary to the mRNA deduced from the following amino acid sequence of the fragment produced by cleaving erythropoietin with cyanogen bromide:

Met-Glu-Val-Gly-Gln-Gln-Ala-Val-Glu-Val-
Trp-Gln-Gly-Leu-Ala-Leu-Leu

and the DNA of the following sequence:

5'CCTCTTCCAGGCATAGAAATTAACTTTGGTGTCTGGGACAGT
GATATTCTCATTCAAGCTGCAGTGTTCAGCACAGCC3'

which is complementary to the mRNA of human erythropoietin, which is obvious from the known cDNA sequence of human erythropoietin (Nature vol. 313, P. 806 (1985)) were synthesized. By colony hybridization using these DNAs as probes, the cDNA library was screened to select the clones positive in hybridization. A plasmid (named as p58-A20) was isolated from a hybridization positive clone and sequencing of the cDNA was conducted to confirm that human erythropoietin was encoded by the cDNA.

The nucleotide sequence of the cDNA obtained from this clone is shown in Fig. 1.

Example 1

(1) Construction of an expression vector

The plasmid p58-A20 containing the human erythropoietin cDNA shown in Fig. 1 (1 $\mu$g) was digested with 6 units of Bst EII, and the DNA was recovered by phenol/chloroform extraction and ethanol precipitation. The DNA was incubated at 37°C for 30 minutes in 50 $\mu$l of a reaction mixture containing 1 mM each of deoxynucleotide triphosphates, 0.1 mM EDTA, 20 mM NaCl, 7 mM $MgCl_2$, 7 mM Tris-HCl (pH 7.5) and 8 units of DNA polymerase I Klenow fragment to convert the sticky end to blant end. The DNA was recovered by phenol/chloroform extraction and ethanol precipitation, and ligated with 5'-end phosphorylated Bgl II linker (Takara shuzo) (0.3 $\mu$g) at 4°C overnight in 50 $\mu$l of a reaction mixture containing 50 mM Tris-HCl (pH 7.5), 10 mM $MgCl_2$, 10 mM dithiothreitol, 1 mM ATP and 250 units of T4 DNA ligase.

Bgl II linker:

5'pCAGATCTG
GTCTAGACp5'

After phenol/chloroform extraction and ethanol precipitation, the DNA was digested with 10 units of Bgl II and recovered by phenol/chloroform extraction and ethanol precipitation. pKSV10 (Pharmacia P-L Biochemicals) (2 $\mu$g) was digested with Bgl II and treated with 20 units of E. coli alkaline phosphatase followed by phenol/chloroform extraction and ethanol precipitation. The resulting Bgl II-digested pKSV10 and the Bgl II fragment of the human erythropoietin cDNA were ligated with 250 units of T4 DNA ligase at 16°C for 3 hours. The reaction mixture was used to transform E. coli HB101 (ATCC 33694). Plasmid DNAs were isolated from the ampicillin-resistant colonies, and analyzed to obtain the DNA with the structure shown as pKSVcEPO in Fig. 2. The insertion of the human erythropoietin cDNA was analyzed by digesting DNAs with Bgl II and its orientation was analyzed by digesting with Kpn I. The pKSVcEPO (2 $\mu$g) was digested with 20 units of Bam HI, and pSV2neo (ATCC 37149) (2 $\mu$g) was digested with 20 units of Bam HI and treated with 20 units of E. coli alkaline phosphatase. The resulting products were ligated with 400 units of T4 DNA ligase at 4°C overnight. The reaction mixture was used to transform E. coli HB101. After plasmid DNAs were isolated from the transformed E. coli resistant to both ampicillin and kanamycin, the structure of the plasmid DNAs was analyzed by digesting with Bgl II and EcoRI and with Bgl II and Hind III to obtain the plasmid with the structure shown in Fig. 3 as pKSVNcEPO.

(2) Transformation of Namalwa cells.

E. coli HB101/pKSVNcEPO harboring the plasmid pKSVNcEPO was grown at 37°C in LB medium containing 1% glycerin, and when the $OD_{600}$ reached to 0.6-0.8 chloramphenicol was added to the medium at a final concentration of 125 $\mu$g/ml and cultivated overnight. The cells from 25 ml of culture were harvested by centrifugation and suspended in 1.25 ml of 20% sucrose and 0.05M Tris-HCl (pH 8.0). To this suspension, 0.25 ml of 5 mg/ml lysozyme solution in 0.25 M Tris-HCl (pH 8.0) was added, and the mixture was kept on ice for 5 minutes. After 0.5 ml of 0.25M EDTA (pH 8.0) was added, the mixture was again kept on ice for 5 minutes and 0.5 ml of 0.05 M Tris-HCl (pH 8.0) was added. The mixture was then incubated at 37°C for 10 minutes. After addition of 10 ml of DME medium containing 10 mM $MgCl_2$ and 10% sucrose, the mixture was incubated at room temperature for 10 minutes. After a pellet of 2 x $10^6$ Namalwa cells (ATCC CRL 1432) was suspended in 5 ml of the resulting mixture, the mixture was centrifuged at 500 x g for 5 minutes and the resulting pellet was suspended in 2 ml of DME medium containing 1g/ml of polyethyleneglycol 1000. The suspension was again centrifuged at 500 x g for 3 minutes to give a pellet, which was then suspended in 7 ml of RPMI 1640 and centrifuged. The pellet was suspended in 2 ml of RPMI 1640 supplemented with 10 $\mu$g/ml of tetracycline and 10% fetal bovine serum, and grown at 37°C under the atmosphere of 5% $CO_2$ for 2 days.

(3) Selection of transformants

The cells grown as mentioned above were inoculated into tissue culture plates with 4 wells at a density of 5 x $10^3$ cells/well, and then grown in RPMI 1640 medium supplemented with 400 μg/ml of G-418 and 10% of fetal bovine serum, while the culture medium was replaced every 3 days. When the cells were grown for 10 - 12 days, 1 - 3 colonies per well were formed. At this point, the erythropoietin activities of the media of the wells were determined by measuring the incorporation of radioactive $^{59}Fe$ into fetal mouse liver cells (Exp. Hematol vol. 3, p. 65 (1975)). With the cells of the wells of which media showed high erythropoietin activity, single colony isolation was performed as follows:

Namalwa cells which were exposed to 6000 rads radiation for 10 minutes were suspended in RPMI 1640 medium supplemented with 400 μg/μl of G-418 and 10% fetal bovine serum and inoculated into plates with 96 wells at a density of 5 x $10^3$ cells/well. The cells to be isolated as a single colony were added to the wells in a ratio of 1 cell per 2 wells. When the cells were cultured for 2 weeks, while replacing the medium every 4 days, growth of the cells was observed in 51 wells out of 91 wells. By measuring the erythropoietin activities of the culture media, the cells producing human erythropoietin in large amounts were selected.

(4) Production of human erythropoietin by the transformed Namalwa cells

The Namalwa cells producing human erythropoietin in large amounts were selected as above and named as Namalwa 2A311, which was inoculated at a density of 1 x $10^6$ cells/ml of RPMI 1640 medium supplemented with 400 μg/ml of G-418 and 10% fetal bovine serum into a 75 $cm^2$ tissue cluture flask and grown at 37°C under the atomosphere of 5% $CO_2$ for 4 days. With the resulting culture medium, 536 units/ml of human erythropoietin was detected.

Example 2

The Namalwa 2A311 obtained in Example 1 was inoculated at a density of 1 x $10^6$ cells/ml in 200 ml of RPMI 1640 medium supplemented with 400 μg/ml of G-418 and 10% fetal bovine serum into a 250 ml spinner vessel and grown at 37°C for 3 days. With the resulting culture medium, 490 units/ml of erythropoietin was detected.

Example 3

The <u>in vivo</u> activities of the erythropoietin in the culture medium of the Namalwa cells obtained in Example 1, the human urine-derived erythropoietin (purchased from TOYOBO CO., LTD., Japan) and the erythropoietin produced by CHO cells (purchased from KIRIN-AMGEN, INC., USA) were compared in accordance with the method using starved rats (Methods in Enzymology vol. 37, P. 109 (1971); RINSHOU KENSA vol. 21, P. 423 (1979)). The experiment was conducted using groups of male Wistar rats aged 5 weeks, each group consisting of 5 rats. During the experiment the rats were kept starved from the first day through the last day. Samples of erythropoietin diluted with saline containing 0.1% bovine serum albumin were subcutaneously injected to the rats at the second and third days followed by intraperitoneal injection of 1mCi of radioactive $^{59}FeCl_3$ at the fourth day. At 18 hours after the $^{59}FeCl_3$ injection, 1 ml each of blood was collected from the hearts of the rats and the radio-activities of the blood samples were measured. The incorporation of radioactive iron into peripheral red blood cells was calculated according to the following formula:

$$\text{Rate of incorporation (\%)} = \frac{\text{count(cpm)/ml of blood x body weight(g) x 5/100}}{\text{count(cpm) of the injected } ^{59}\text{Fe}} \times 100$$

The results are given in Fig. 4, where the dose response curves of the tested erythropoietin are shown. The erythropoietin producted by Namalwa cells was found to have the same <u>in vivo</u> activity as that of the natural erythropoietin derived from human urine, while the erythropoietin produced by CHO cells showed a dose response curve different from that of the erythropoietin derived from human urine.

Example 4

The binding properties of the erythropoietin purified from culture medium of the Namalwa cells obtained in Example 1 to lectins were compared with those of the erythropoietin derived from human urine and CHO cells. Columns (0.2 ml) were packed with Concanavalin A (Con-A) - Sepharose (Pharmacia Chemicals), phytohemagglutinin (PHA-E) - agarose (E Y Laboratories), Lentil lectin (LCA) - agarose (SEIKAGAKU KOGYO) and Peanut lectin (PNA) - agarose (SEIKAGAKU KOGYO), each of which was equilibrated with Dulbecco's phosphate buffered saline containing 0.01% bovine serum. To these columns, 0.5 ml each of Dulbecco's phosphate buffered saline containing 10 units of the erythropoietin sample was applied and the columns were washed with 0.5 ml of the same saline. The amounts of the erythropoietin in 1 ml of the flow through fraction were measured. The recovery of the erythropoietin in the collected buffers is shown in Table 1. As is clear from the Table 1, the binding properties of the erythropoietin produced by Namalwa cells to the lectins are different from those of the erythropoietin produced by CHO cells, but in fair agreement with those of the erythropoietin derived from human urine. This indicates good similarity in structure, particularly in sugar chain structure of the erythropoietin produced by Namalwa cells to the human urine-derived erythropoietin.

Table 1

| The nonbinding percentages of erythropoietin samples to lectins | | | |
|---|---|---|---|
| Lectins | Source of erythropoietin | | |
| | Urine | Namalwa cells | CHO cells |
| Con A | 86.6 | 73.2 | 42.4 |
| PHA-E | 75.4 | 61.1 | 42.9 |
| LCA | 71.1 | 80.6 | 45.2 |
| PNA | 79.6 | 98.8 | 37.8 |

Figure 1 illustrates the nucleotide sequence of the human erythropoietin cDNA and the deduced amino acid sequence of human erythropoietin. The downward arrow indicates Bst EII site and the upward arrow indicates Bgl II site. The underline indicates the first amino acid of human erythropoietin.

Figure 2 illustrates a schematic diagram of the construction of pKSVcEPO from pKSV10 and p58-A20.

Figure 3 illustrates a schematic diagram of the construction of pKSVNcEPO from pKSVcEPO and pSV2neo.

Figure 4 illustrates the in vivo activities of the erythropoietin produced by Namalwa cells and CHO cells and derived from human urine.

## Claims

1. A process for producing human erythropoietin which comprises culturing Namalwa cells transformed with DNA encoding human erythropoietin to produce human erythropoietin in a culture medium and separating the resulting erythropoietin from the culture medium.

2. A Namalwa cell containing exogenous DNA encoding human erythropoietin and able to produce erythropoietin.

3. Plasmid pKSVNcEPO as illustrated in Fig. 3.

4. E. Coli harboring plasmid pKSVNcEPO as illustrated in Fig 3.

## Revendications

1. Procédé de production d'érythropoiétine humaine, qui consiste à cultiver des cellules de Namalwa transformées par de l'ADN codant pour de l'érythropoiétine humaine pour produire de l'érythropoiétine dans un milieu de culture et à séparer l'érythropoiétine obtenue du milieu de culture.

2. Cellule de Namalwa contenant de l'ADN exogène codant pour l'érythropoiétine humaine et susceptible de produire de l'érythropoiétine.

3. Le plasmide pKsVNcEPO tel qu'illustré à la figure 3.

4. E. Coli servant d'hôte au plasmide pKSVNcEPO tel qu'illustré à la figure 3.

**Patentansprüche**

1. Verfahren zur Herstellung von menschlichem Erythropoietin, umfassend die Züchtung von Namalwa-Zellen, die mit für menschliches Erythropoietin codierender DNA transformiert sind, so daß menschliches Erythropoietin in einem Kulturmedium produziert wird, und Abtrennung des erhaltenen Erythropoietins aus dem Kulturmedium.

2. Namalwa-Zelle, enthaltend exogene, für menschliches Erythropoietin codierende DNA, die fähig ist, Erythropoietin zu produzieren.

3. Plasmid pKSVNcEPO gemäß der Beschreibung in Fig. 3.

4. E. coli, enthaltend das Plasmid pKSVNcEPO gemäß der Beschreibung in Fig. 3.

# FIG. 1

```
                                 ↓
AGCTTCCCGGGATGAGGGCCCCCGGTGTGG TCACCCGGCGCGCCCCAGGTCGCTGAGGGA   60

CCCCGGCCAGGCGCGGAGATGGGGGTGCAC GAATGTCCTGCCTGGCTGTGGCTTCTCCTG  120
                    MetGlyValHis GluCysProAlaTrpLeuTrpLeuLeuLeu

TCCCTGCTGTCGCTCCCTCTGGGCCTCCCA GTCCTGGGCGCCCCACCACGCCTCATCTGT  180
SerLeuLeuSerLeuProLeuGlyLeuPro ValLeuGlyAlaProProArgLeuIleCys

GACAGCCGAGTCCTGGAGAGGTACCTCTTG GAGGCCAAGGAGGCCGAGAATATCACGACG  240
AspSerArgValLeuGluArgTyrLeuLeu GluAlaLysGluAlaGluAsnIleThrThr

GGCTGTGCTGAACACTGCAGCTTGAATGAG AATATCACTGTCCCAGACACCAAAGTTAAT  300
GlyCysAlaGluHisCysSerLeuAsnGlu AsnIleThrValProAspThrLysValAsn

TTCTATGCCTGGAAGAGGATGGAGGTCGGG CAGCAGGCCGTAGAAGTCTGGCAGGGCCTG  360
PheTyrAlaTrpLysArgMetGluValGly GlnGlnAlaValGluValTrpGlnGlyLeu

GCCCTGCTGTCGGAAGCTGTCCTGCGGGGC CAGGCCCTGTTGGTCAACTCTTCCCAGCCG  420
AlaLeuLeuSerGluAlaValLeuArgGly GlnAlaLeuLeuValAsnSerSerGlnPro

TGGGAGCCCCTGCAGCTGCATGTGGATAAA GCCGTCAGTGGCCTTCGCAGCCTCACCACT  480
TrpGluProLeuGlnLeuHisValAspLys AlaValSerGlyLeuArgSerLeuThrThr

CTGCTTCGGGCTCTGGGAGCCCAGAAGGAA GCCATCTCCCCTCCAGATGCGGCCTCAGCT  540
LeuLeuArgAlaLeuGlyAlaGlnLysGlu AlaIleSerProProAspAlaAlaSerAla

GCTCCACTCCGAACAATCACTGCTGACACT TTCCGCAAACTCTTCCGAGTCTACTCCAAT  600
AlaProLeuArgThrIleThrAlaAspThr PheArgLysLeuPheArgValTyrSerAsn

TTCCTCCGGGGAAAGCTGAAGCTGTACACA GGGGAGGCCTGCAGGACAGGGGACAGATGA  660
PheLeuArgGlyLysLeuLysLeuTyrThr GlyGluAlaCysArgThrGlyAspArg***

CCAGGTGTGTCCACCTGGGCATATCCACCA CCTCCCTCACCAACATTGCTTGTGCCACAC  720

CCTCCCCCGCCACTCCTGAACCCCGTCGAG GGGCTCTCAGCTCAGCGCCAGCCTGTCCCA  780

TGGACACTCCAGTGCCACCAATGACATCTC AGGGGCCAGAGGAACTGTCCAGAGAGCAAC  840

TCTGAGATCTAAGGATGTCACAGGGCCAAC TTGAGGGCCCAGAGCAGGAAGCATTCAGAG  900
     ↑
AGCAGCTTTAAACTCAGGGACAGACCCATG CTGGGAAGACGCCTGAGCTCACTCGGCACC  960

CTGCAAAATTTGATGCCAGGACACGCTTTG GAGGCGATTTACCTGTTTTCGCACCTACCA 1020

TCAGGGACAGGATGACCTGGAGAACTTAGG TGGCAAGCTGTGACTTCTCCAGGTCTCACG 1080

GGCATGGGCACTCCCTTGGTGGCAAGAGCC CCCTTGACACCGGGGTGGTGGGAACCATGA 1140

AGACAGGATGGGGGCTGGCCTCTGGCTCTC ATGGGGTCCAACTTTTGTGTATTCTTCAAC 1200

CTCATTGACAAGAACTGAAACCACCAAAAA AAAAAAAAAAAAAAA                1260
```

FIG. 2

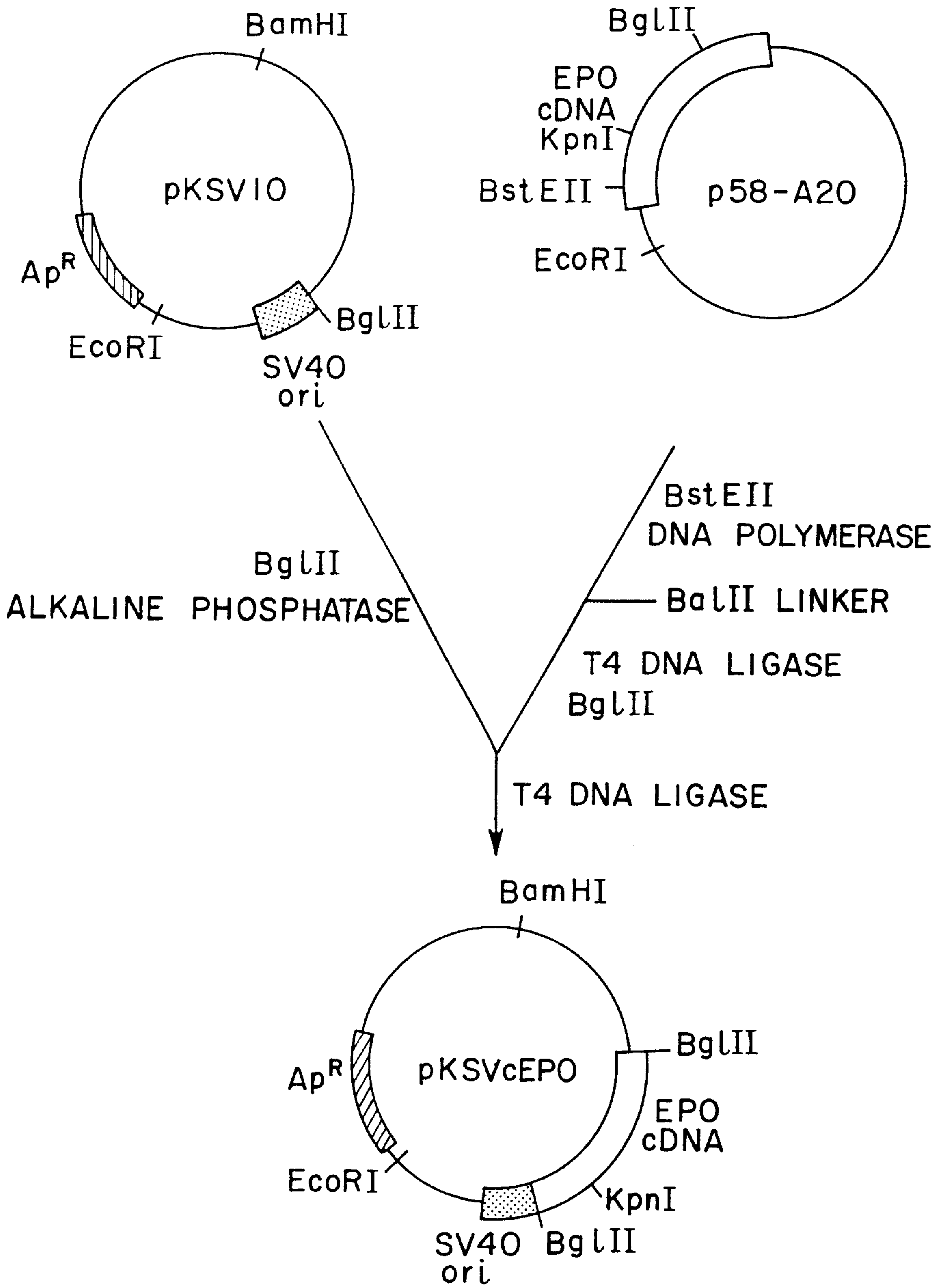
BamHI
pKSV10
ApR
EcoRI
SV40 ori
BglII
BglII
EPO cDNA
KpnI
BstEII
p58-A20
EcoRI
BglII
ALKALINE PHOSPHATASE
BstEII
DNA POLYMERASE
BalII LINKER
T4 DNA LIGASE
BglII
T4 DNA LIGASE
BamHI
ApR
pKSVcEPO
EcoRI
BglII
EPO cDNA
KpnI
SV40 ori
BglII

FIG. 3

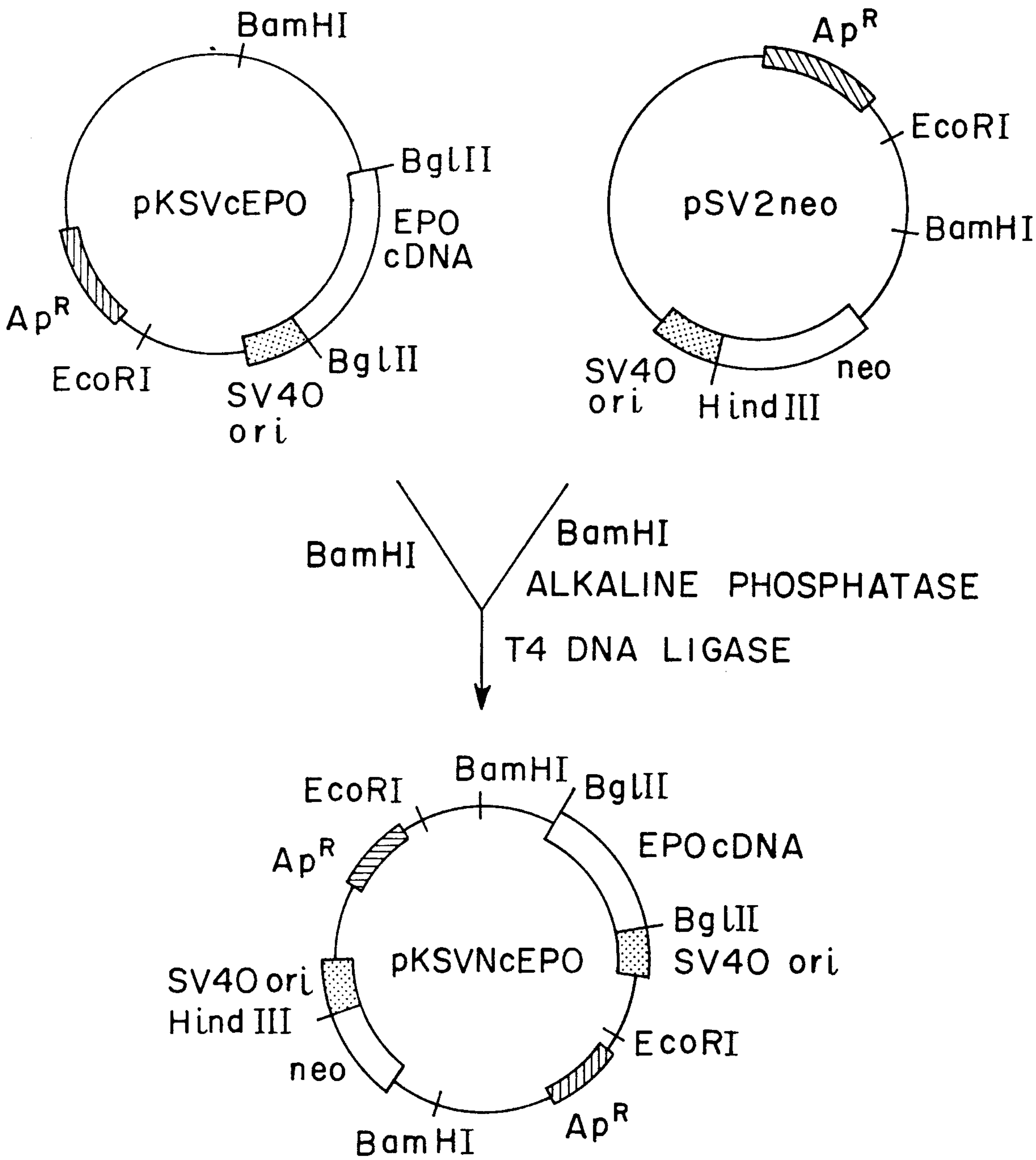
BamHI
BglII
pKSVcEPO
EPO cDNA
ApR
EcoRI
BglII
SV40 ori
ApR
EcoRI
pSV2neo
BamHI
SV40 ori
neo
HindIII
BamHI
BamHI
ALKALINE PHOSPHATASE
T4 DNA LIGASE
EcoRI
BamHI
BglII
EPOcDNA
ApR
BglII
SV40 ori
SV40 ori
pKSVNcEPO
HindIII
EcoRI
neo
BamHI
ApR

FIG. 4

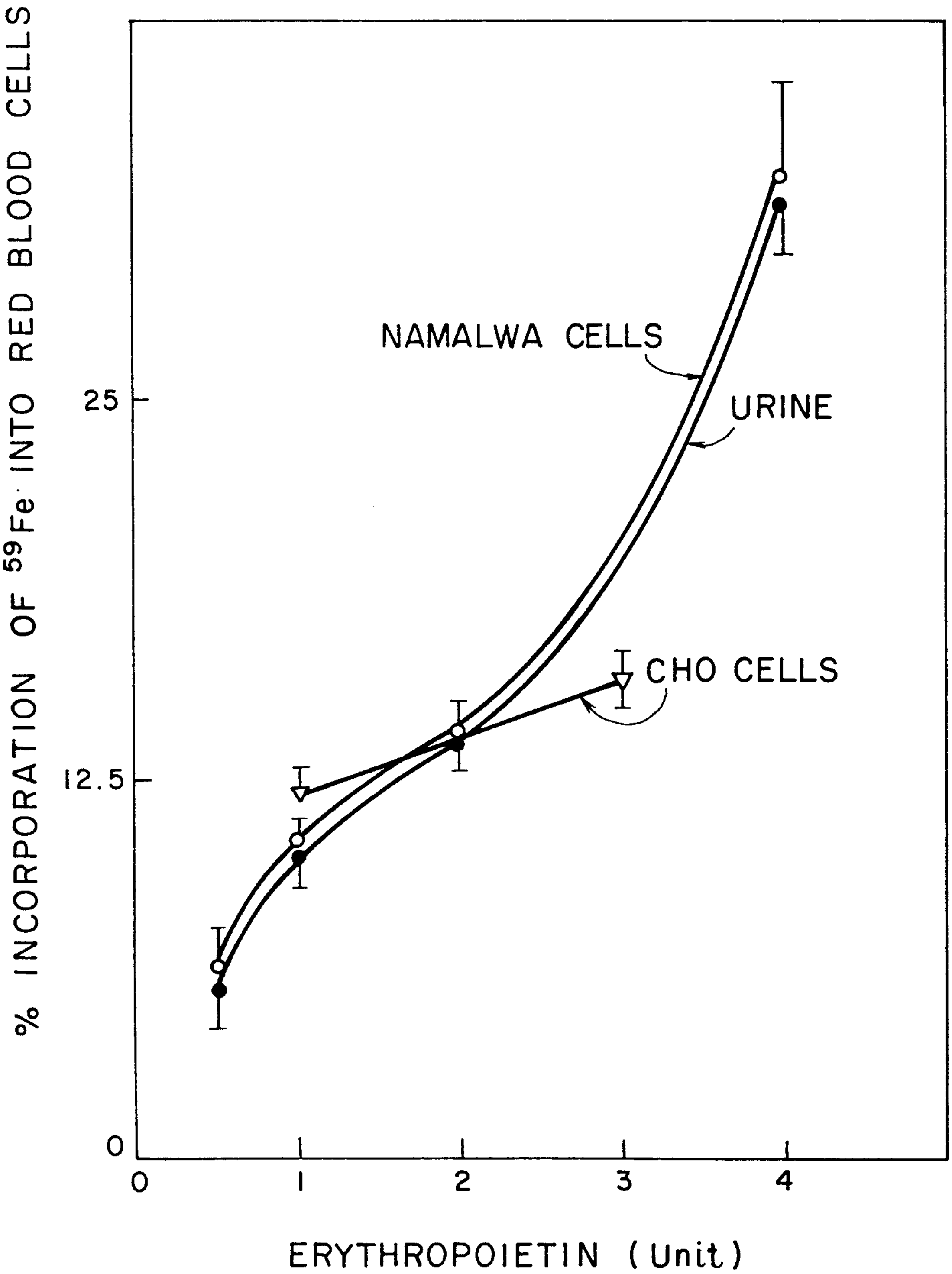
% INCORPORATION OF $^{59}$Fe· INTO RED BLOOD CELLS
25
12.5
0
NAMALWA CELLS
URINE
CHO CELLS
0
1
2
3
4
ERYTHROPOIETIN (Unit)